# EUROPEAN PATENT SPECIFICATION

(11) **EP 3 444 356 B1**
(45) Date of publication and mention of the grant of the patent: **17.02.2021**
(21) Application number: 16890126.2
(22) Date of filing: 25.03.2016
(51) Int. Cl.: C12Q 1/02, A61K 31/713, A61K 48/00, A61P 17/08, A61P 17/10, A61P 17/14, C12N 15/09

(54) **METHOD FOR ASSESSING OR SELECTING SEBACEOUS-GLAND- OR HAIR-FOLLICLE-SELECTIVE ANDROGEN RECEPTOR ACTIVITY CONTROLLING AGENT**
VERFAHREN ZUR BEURTEILUNG ODER AUSWAHL EINES FÜR TALGDRÜSEN ODER HAARFOLLIKEL SELEKTIVEN ANDROGENREZEPTORAKTIVITÄTSREGLERS
PROCÉDÉ D'ÉVALUATION OU DE CHOIX DE RÉGULATEUR D'ACTIVITÉ DE RÉCEPTEUR D'ANDROGÈNE DE GLANDE SÉBACÉE OU DE FOLLICULE PILEUX AU CHOIX

(43) Date of publication of application: 20.02.2019
(73) Proprietor: Kao Corporation, Chuo-ku Tokyo 103-8210 (JP)
(72) Inventor: INOUE, Takayoshi, Tochigi 321-3497 (JP); HACHIYA, Akira, Tochigi 321-3497 (JP); KATO, Arisa, Tochigi 321-3497 (JP)
(74) Representative: Vossius & Partner Patentanwälte Rechtsanwälte mbB
(86) International application number: PCT/JP2016/059553
(87) International publication number: WO 2017/163391

(56) References cited:
- FR-A1- 2 854 243
- RANDALL. VALERIE ANNE: "Androgens and hair growth", Dermatology therapy, vol. 21, 2008, pages 314-328, XP055426699,
- GILTAY. E.J et al.: "Effects of sex steroid deprivation/administration on hair growth and skin sebum production in transsexual males and females", Journal of clinical endocrinology and metabolism, vol. 85, 2000, pages 2913-2921, XP055426700,
- ROGENBERGER CHRISTIAN et al.: "Upregulation of hypoxia-inducible factors in normal and psoriatic skin", Journal of Investigative Dermatology, vol. 127, 2007, pages 2445-2452, XP055426701,
- TAKAKAZU MITANI et al.: "Teisanso Signal ni yoru Androgen Juyotai Signal no Chosetsu", Journal of Japanese Biochemical Society, vol. 146, 25 September 2009 (2009-09-25), page 4P-247, XP009516477, ISSN: 0021-924X

## Description

### Field

The present disclosure relates to in vitro methods of identifying an agent for regulating activity of an androgen receptor in a sebaceous gland- or hair follicle-selective manner.

### Background

Androgen receptor (AR) is a member of the steroid hormone receptor subfamily, which includes mineralocorticoid receptor (MR), progesterone receptor (PR), estrogen receptor (ER), and glucocorticoid receptor (GR). Endogenous steroid androgen (such as testosterone and 5α-dihydrotestosterone (DHT)), which is a group of major circulating hormones, promotes the development of secondary sexual characteristics and plays important roles in the modulation of various physiological processes. As for the roles of androgen in skin, it has been reported that dehydroepiandrosterone (DHEA) improved skin condition on aged people when administered by oral route and that DHEA increased the level of sebum in menopausal females who usually have decreased level of sebum when topically applied onto the skin (Non Patent Literature 1). In addition, when an androgen receptor repressor was administered, the androgen receptor repressor promoted growth of hair at the top of the head but repressed growth of hair except at the top of the head and further decreased the level of sebum secretion. On the other hand, when an androgen was administered, the androgen repressed growth of hair at the top of the head but promoted growth of hair except at the top of the head and further increased the level of sebum secretion. These results indicate that androgen can comprehensively regulate characteristics of sebaceous glands and hair (Non Patent Literature 2, 3). Some agents which can improve acne, alopecia, or hypertrichosis by repressing activity of androgen receptors have so far been developed (Non Patent Literature 4, 5).

However, repression of androgen receptors activity can cause side effects due to gonadal hypofunction such as menstrual irregularity, breast hypertrophy in males, testicular atrophy, and sexual dysfunction (hypofunction of gonad including testis, mammary gland, uterus, and ovary). For this reason, the use of androgen receptor activity repressors is not approved for improving the conditions of sebaceous glands and hair as described above in the United States. Therefore, there remains a need for androgen receptor activity regulators selective to sebaceous glands and hair follicle tissues without any side effect due to gonadal hypofunction.

Hypoxia Inducible Factor 1, alpha subunit (HIF1α) is a molecule whose expression is induced by hypoxia. HIF1α acts as a master modulator of cellular hypoxic response by activating transcription of many genes involved in energy metabolism, vascularization, apoptosis, and the like. HIF1α was reported to have an effect to repress cell death of neutrophils due to apoptosis in immune system (Non Patent Literature 6). HIF1α was also reported to control wound healing and barrier function in skin (Non Patent Literature 7). Moreover, HIF1α was reported to induce upregulation of glycolytic enzymes in a hypoxic environment (Non Patent Literature 8). HIF1α was further reported to be expressed in human sebaceous glands in which oxygen concentration is very low and ranges from 0.1% to 1.3% (Non Patent Literature 9, 10). Furthermore, a method for identifying compounds for treatment of alopecia by measuring the level of hypoxia-inducible factor-1 was reported (FR 2854243 A1). However, the relationship between androgen receptors in sebaceous glands and hair follicles and HIF1α or glycolytic enzymes is unknown.
[Non Patent Literature 1] Maturitas, 2008, 59: 174-181
[Non Patent Literature 2] Dermatol Ther, 2008, 21: 314-328
[Non Patent Literature 3] J Clin Endocrinol Metab, 2000, 85: 2913-2921
[Non Patent Literature 4] Cochrane database of systematic reviews, 2001, CD000194
[Non Patent Literature 5] Br J Dermatol, 2005, 152: 466-473
[Non Patent Literature 6] J Exp Med, 2005, 201: 105-115
[Non Patent Literature 7] J Invest Dermatol, 2011, 131: 1793-1805
[Non Patent Literature 8] Mol Cell, 2010, 40: 294-309
[Non Patent Literature 9] J Invest Dermatol, 2006, 126: 2596-2606
[Non Patent Literature 10] J Invest Dermatol, 2007, 127: 2445-2452

### Summary

The present disclosure provides an in vitro method of evaluating or selecting an androgen receptor activity regulator selective to sebaceous glands or hair follicles, the method comprising:
(A) applying a test agent to cells from sebaceous glands or hair follicles under a hypoxic condition;
(B) measuring HIF1α activity in the cells;
(C) comparing the HIF1α activity measured in the (B) with HIF1α activity in a control group; and
(D) determining a regulatory effect of the test agent on the HIF1α activity based on the result in the (C).

The invention relates to the embodiments defined in the claims. In particular, the method as described in the context of the invention relates to an "in *vitro"* method. In this regard, the present invention provides an in vitro method of evaluating or selecting an androgen receptor activity regulator selective to sebaceous glands or hair follicles, the method comprising:
(A) applying a test agent to cells from sebaceous glands or hair follicles under a hypoxic condition;
(B') measuring activity of a factor induced by HIF1α in the cells, wherein the factor induced by HIF1α is a glycolytic enzyme;
(C') comparing the activity measured in the (B') with activity of the factor in a control group; and
(D') determining a regulatory effect of the test agent on the activity of the factor based on the result in the (C').

Disclosed herein is also an androgen receptor activity repressor selective to sebaceous glands or hair follicles, a sebum secretion repressor, an agent for preventing or improving acne, an agent for preventing or improving alopecia, or an agent for preventing or improving hypertrichosis which comprises, as an active ingredient, at least one selected from the group consisting of siRNAs for each of HIF1α gene, ENOl gene, LDHA gene, PGK1 gene, GPI gene, and HK1 gene.

Disclosed herein is also at least one selected from the group consisting of siRNAs for each of HIF1α gene, ENOl gene, LDHA gene, PGK1 gene, GPI gene, and HK1 gene for the manufacture of an androgen receptor activity repressor selective to sebaceous glands or hair follicles, a sebum secretion repressor, an agent for preventing or improving acne, an agent for preventing or improving alopecia, or an agent for preventing or improving hypertrichosis.

The present invention also provides non-therapeutic use of at least one selected from the group consisting of siRNAs for each of HIF1α gene, ENOl gene, LDHA gene, PGK1 gene, GPI gene, and HK1 gene for use in repressing sebum secretion.

The present invention also provides at least one selected from the group consisting of siRNAs for each of HIF1α gene, ENOl gene, LDHA gene, PGK1 gene, GPI gene, and HK1 gene for use in the prevention or improvement of acne, the prevention or improvement of alopecia, or the prevention or improvement of hypertrichosis.

### Brief Description of the Drawings

[Figure 1] Figure 1 shows effect of the expression of glycolytic enzymes on AR activities.
[Figure 2] Figure 2 shows effect of HIF1α on the expression of glycolytic enzymes.
[Figure 3] Figure 3 shows effect of HIF1α on the AR activities in sebaceous gland cells (SZ95).
[Figure 4] Figure 4 shows effect of HIF1α on the AR activities in prostate cancer cells (LNCaP).
[Figure 5] Figure 5 shows HIF1α activities altered by retinoic acid.

### Detailed Description

As used herein, "Hypoxia Inducible Factor 1, alpha subunit (HIF1α)" refers to a protein which is registered in OMIM under No. 603348 and in Genbank under Accession No. NG_029470.1 or a homolog, paralog, or ortholog thereof. Preferably, as used herein "HIF1α" refers to a protein which consists of the amino acid sequence as set forth in SEQ ID NO: 2 or an amino acid sequence having at least 90% identity to the amino acid sequence as set forth in SEQ ID NO: 2 and acts as a transcription factor whose expression is induced by hypoxia. HIF1α is transported into nucleus followed by forming a heterocomplex with other protein and binding a site called Hypoxia Response Element (HRE) on DNA to induce various hypoxic responses in cells. Factors induced by HIF1α include glycolytic enzymes. Examples of glycolytic enzymes include enolase 1, lactate dehydrogenase A, phosphoglycerate kinase 1, glucose-6-phosphate isomerase, and hexokinase 1, which are coded by ENO1, LDHA, PGK1, GPI, and HK1 genes respectively.

Accordingly, "HIF1α activity" as used herein refers to transcriptional activity of HIF1α. The transcriptional activity of HIF1α can be measured by determining a binding level of HIF1α to HRE, an expression level of HIF1α gene, or an expression level of HIF1α protein.

As used herein, "HIF1α gene" is a gene which encodes the "HIF1α". As used herein, "HIF1α gene" preferably refers to a gene which is registered in Genbank under Accession No. NG_029470.1 or a homolog, paralog, or ortholog thereof and more preferably refers to a polynucleotide which consists of the nucleotide sequence as set forth in SEQ ID NO: 1 or a nucleotide sequence having at least 90% identity to the nucleotide sequence as set forth in SEQ ID NO: 1 and encodes a protein which acts as a transcription factor whose expression is induced by hypoxia.

As used herein, a "factor induced by HIF1α" (hereinafter also referred as "HIF1α inducible factor") refers to a factor whose expression or activation is induced by HIF1α and preferably refers to at least one of glycolytic enzymes selected from the group consisting of enolase 1, lactate dehydrogenase A, phosphoglycerate kinase 1, glucose-6-phosphate isomerase, and hexokinase 1. The genes encoding these glycolytic enzymes include ENO1, LDHA, PGK1, GPI, and HK1.

ENO1 is a gene registered in OMIM under No. 172430 and in Genbank under Accession No. NG_029470.1. Preferably, as used herein "ENOl" refers to a polynucleotide which consists of the nucleotide sequence as set forth in SEQ ID NO: 3 or a nucleotide sequence having at least 90% identity to the nucleotide sequence as set forth in SEQ ID NO: 3 and encodes an enzyme which converts 2-phosphoglyceric acid to phosphoenolpyruvic acid.

LDHA is a gene registered in OMIM under No. 150000 and in Genbank under Accession No. NG_008185.1. Preferably, as used herein "LDHA" refers to a polynucleotide which consists of the nucleotide sequence as set forth in SEQ ID NO: 4 or a nucleotide sequence having at least 90% identity to the nucleotide sequence as set forth in SEQ ID NO: 4 and encodes an enzyme which converts L-lactate and NAD to pyruvate and NADH.

PGK1 is a gene registered in OMIM under No. 311800 and in Genbank under Accession No. NG_008862.1. Preferably, as used herein "PGK1" refers to a polynucleotide which consists of the nucleotide sequence as set forth in SEQ ID NO: 5 or a nucleotide sequence having at least 90% identity to the nucleotide sequence as set forth in SEQ ID NO: 5 and encodes an enzyme which converts 1,3-diphosphoglycerate to 3-phosphoglycerate.

GPI is a gene registered in OMIM under No. 172400 and in Genbank under Accession No. NG_012838.2. Preferably, as used herein "GPI" refers to a polynucleotide which consists of the nucleotide sequence as set forth in SEQ ID NO: 6 or a nucleotide sequence having at least 90% identity to the nucleotide sequence as set forth in SEQ ID NO: 6 and encodes an enzyme which converts glucose-6-phosphate to fructose-6-phosphate.

HK1 is a gene registered in OMIM under No. 142600 and in Genbank under Accession No. NG_012077.1. Preferably, as used herein "HK1" refers to a polynucleotide which consists of the nucleotide sequence as set forth in SEQ ID NO: 7 or a nucleotide sequence having at least 90% identity to the nucleotide sequence as set forth in SEQ ID NO: 7 and encodes an enzyme which converts glucose to glucose-6-phosphate.

As used herein, "activity of a factor induced by HIF1α (or an HIF1α inducible factor)" preferably refers to activity of at least one glycolytic enzyme selected from the group consisting of enolase 1, lactate dehydrogenase A, phosphoglycerate kinase 1, glucose-6-phosphate isomerase, and hexokinase 1.

As used herein, "at least 90% identity" in the context of an amino acid sequence and a nucleotide sequence refers to identity of 90% or more, preferably 95% or more, more preferably 98% or more, and further preferably 99% or more.

As used herein, an identity of a nucleotide sequence and an amino acid sequence is calculated using the Lipman-Pearson method (Science, 1985, 227: 1435-1441). Specifically, the identity can be determined using a homology analysis (Search homology) program of genetic information processing software Genetyx-Win (Ver.5.1.1; Software Development Corporation Limited) with the unit size to compare (ktup) being set to 2.

As used herein, "alopecia" refers to a condition characterized by decrease in hair in the head and thinning hair while "hypertrichosis" refers to a condition characterized by increased hair due to thickening or elongating of whiskers and hairs in hand, foot, trunk, and other body parts.

As used herein, "preventing" refers to prevention, suppression, or delay of development of a disease or condition in an individual or refers to decrease in risk of development of a disease or condition in an individual. As used herein, "improving" refers to reversal of a disease or condition, prevention, suppression, or delay of deterioration of a disease or condition, or reversal, prevention, suppression, or delay of progression of a disease or condition.

As used herein, "non-therapeutic" refers to the concept without including medical practices, that is, methods of operating, treating, or diagnosing human, and more specifically refers to the concept without including methods of operating, treating, or diagnosing human which are performed by a doctor or a medical practitioner or a person receiving an instruction from a doctor.

The present disclosure provides a method of identifying an agent for regulating activity of an androgen receptor in a sebaceous gland- or hair follicle-selective manner without concern of side effects via androgen receptors in gonads.

The present inventors earnestly investigated to identify a molecule contributing to activity of androgen receptors in a sebaceous gland- and hair follicle-selective manner. The present inventors consequently found that expression of HIF1α in sebaceous glands and hair follicles is markedly higher than the expression of HIF1α in gonads and that HIF1α can positively regulate activity of androgen receptors under a hypoxic condition in sebaceous gland cells and hair follicles. In other words, the present inventors found that an androgen receptor activity regulator which can regulate activity of an androgen receptor in a sebaceous gland- and hair follicle-selective manner can be evaluated or selected by utilizing an activity level of HIF1α in cells under a hypoxic condition as an indicator.

As described herein, an agent for regulating activity of an androgen receptor in a sebaceous gland- and hair follicle-selective manner can be identified. An androgen receptor activity regulator identified by using the method as described herein can be used to control a condition associated with sebaceous glands and hair, for example, to control sebum secretion, to prevent or improve acne, to prevent or improve alopecia, to prevent or improve hypertrichosis, and to prevent or improve senile xerosis. An androgen receptor activity regulator identified by using the method as described herein has low risk of side effects caused by androgen receptors in gonads and can be used safely.

As described in the Examples below, the expression level of HIF1α in sebaceous glands and hair follicles was markedly higher than the expression level of HIF1α in gonads (Example 1). The present inventors found that HIF1α positively regulated the activity of androgen receptors in sebaceous glands under a hypoxic condition (Example 4). The present inventors also found that a factor whose expression is induced by the activation of HIF1α positively regulated the activity of androgen receptors in sebaceous glands (Example 2). On the other hand, it is well known that androgen is involved in sebum secretion and hair growth through action to sebaceous glands and hair follicles (e.g., Non Patent Literature 1, 2). Accordingly, an agent which can alter the activity of HIF1α or an HIF1α inducible factor can alter the activity of androgen receptors in a sebaceous gland cell- and hair follicle cell-selective manner and thus can prevent or improve various conditions resulted from activation or repression of androgen receptors activity in sebaceous gland cells or hair follicle cells including excess or decreased secretion of sebum, acne, hair loss, and hypertrichosis.

Provided herein is a method of evaluating a regulatory effect of various agents on activity of androgen receptors by utilizing an activity level of HIF1α or an HIF1α inducible factor as an indicator or a method of selecting an androgen receptor activity regulator selective to sebaceous glands or hair follicles or a regulator of a condition associated with sebaceous glands and hair, including a sebum secretion regulator, an agent for preventing or improving acne, an agent for preventing or improving alopecia, an agent for preventing or improving hypertrichosis, and an agent for preventing or improving senile xerosis, based on the evaluation. The evaluation or selection method as described herein may be performed in vitro or ex vivo.

Accordingly, disclosed herein is a method of evaluating or selecting an androgen receptor activity regulator selective to sebaceous glands or hair follicles, the method comprising:
(A) applying a test agent to cells from sebaceous glands or hair follicles under a hypoxic condition;
(B) measuring HIF1α activity in the cells;
(C) comparing the HIF1α activity measured in the (B) with HIF1α activity in a control group; and
(D) determining a regulatory effect of the test agent on the HIF1α activity based on the result in the (C).

Disclosed herein is also a method of evaluating or selecting a sebum secretion regulator, an agent for preventing or improving acne, an agent for preventing or improving alopecia, an agent for preventing or improving hypertrichosis, or an agent for preventing or improving senile xerosis, the method comprising (A) to (D) as described above.

Further disclosed herein is a method of evaluating or selecting an androgen receptor activity regulator selective to sebaceous glands or hair follicles, the method comprising:
(A) applying a test agent to cells from sebaceous glands or hair follicles under a hypoxic condition;
(B') measuring activity of a factor induced by HIF1α in the cells;
(C') comparing the activity measured in the (B') with activity of the factor in a control group; and
(D') determining a regulatory effect of the test agent on the activity of the factor based on the result in the (C'). In a preferred embodiment, the factor induced by HIF1α is at least one of glycolytic enzymes selected from the group consisting of enolase 1, lactate dehydrogenase A, phosphoglycerate kinase 1, glucose-6-phosphate isomerase, and hexokinase 1.

Still another embodiment as described herein is a method of evaluating or selecting a sebum secretion regulator, an agent for preventing or improving acne, an agent for preventing or improving alopecia, an agent for preventing or improving hypertrichosis, or an agent for preventing or improving senile xerosis, the method comprising (A) and (B') to (D') as described above.

Test agents used in the methods of the present disclosure are not especially limited as long as the test agents are desired to be used as an androgen receptor activity regulator, a sebum secretion regulator, an agent for preventing or improving acne, an agent for preventing or improving alopecia, an agent for preventing or improving hypertrichosis, or an agent for preventing or improving senile xerosis. The test agents may be naturally occurring agents or artificially synthesized agents including chemically or biologically synthesized agents and may be also compounds or compositions or mixtures.

The "cells from sebaceous glands or hair follicles" used in the methods as described herein include sebaceous gland cells or hair follicle cells isolated from mammals, or cultures of the sebaceous gland cells or hair follicle cells. The cultures of sebaceous gland cells or hair follicle cells include a sebaceous gland cell line, a hair follicle cell line, and a tissue or organ culture of skin and hair follicles (such as a culture of isolated hair follicles, an organ culture of hair follicles, and three-dimensionally cultured skin) and are preferably cells of an established human sebaceous gland cell line. Examples of human sebaceous gland cell lines include, but are not limited to, an immortalized sebocyte cell line from human (such as DSM ACC2383 or SZ95 and SEB-1) (see JP-A-2002-535984, J Invest Dermatol, 1999, 113: 1011-1120, and J Invest Dermatol, 2003, 120: 905-914).

Mammals from which the cells from sebaceous glands or hair follicles are derived include, but are not limited to, for example, human, mouse, rat, hamster, guinea pig, rabbit, cat, dog, pig, and monkey. The mammal is preferably human.

In the methods as described herein, a test agent is applied to the cells from sebaceous glands or hair follicles under a hypoxic condition. A specific example of the procedure can include seeding the cells in a cell medium to which the test agent was previously added and culturing the cells under a hypoxic condition; adding a test agent to a cell medium in which the cells are cultured under a hypoxic condition and further culturing the cells under a hypoxic condition; and adding a test agent to a cell medium in which the cells are cultured at a normal oxygen concentration and further culturing the cells under a hypoxic condition. When the cells are from an organ culture or tissue culture, the test agent may be directly administered to the organ or tissue instead of addition to the cell medium.

In the methods as described herein, the expression of HIF1α or an HIF1α inducible factor is induced in the cells from sebaceous glands or hair follicles by culturing the cells under a hypoxic condition. Accordingly, a "hypoxic condition" as used herein may be a condition of oxygen concentration which can induce HIF1α expression in cells and preferably is a condition of a concentration of oxygen in the cell culture atmosphere of about 5% or less and more preferably of from about 0.1% to about 5%. The hypoxic condition can be achieved by an incubator equipped with an oxygen concentration controller. Such incubators are known and commercially available in various types (such as BIONIX hypoxic cell culture kit; SUGIYAMA-GEN CO., LTD and AnaeroPack-Anaero; MITSUBISHI GAS CHEMICAL COMPANY, INC.).

Next, in the methods as described herein, the activity of HIF1α or an HIF1α inducible factor is measured in the cells from sebaceous glands or hair follicles. Preferably, the activity of HIF1α is measured by determining a binding level of HIF1α to HRE, an expression level of HIF1α gene, or an expression level of HIF1α protein. Among them, the measurement by determining the binding level of HIF1α to HRE is preferable because of its simplicity and rapidity. The activity of an HIF1α inducible factor is measured by determining an expression level of the gene of the HIF1α inducible factor or an expression level of the protein coded by the gene. Preferably, the activity of an HIF1α inducible factor is measured by determining an expression level of at least one gene selected from the group consisting of ENOl, LDHA, PGK1, GPI, and HK1 or a level of expression or enzymatic activity of at least one enzyme selected from the group consisting of enzymes coded by these genes.

The binding activity level of HIF1α to HRE may be measured according to any method known in the art. For example, expression of a marker gene (e.g., luciferase gene) which is operably linked downstream to HRE, for example, luciferase activity may be determined in cells into which the marker gene has been introduced. The expression level of HIF1α or an HIF1α inducible factor can be determined by measuring the expression level of the gene of HIF1α or the HIF1α inducible factor or the expression level of the protein of HIF1α or the HIF1α inducible factor. The expression level of the gene of HIF1α or the HIF1α inducible factor can be determined by quantifying mRNA transcribed from the gene. The quantification of mRNA can be performed using real-time RT-PCR, RNase protection assay, or Northern blot analysis. The expression level of the protein of HIF1α or the HIF1α inducible factor can be determined using a conventional immunoassay, for example, RIA, EIA, ELISA, bioassay, proteome analysis, Western blot analysis, and the like. Among them, real-time RT-PCR is low-cost and simple.

In the methods as described herein, activity of HIF1α or an HIF1α inducible factor in the cells from sebaceous glands or hair follicles in a subject of interest (test group) is compared with activity in a control group. The cells in the control group includes the same cells from sebaceous glands or hair follicles as in a test group which are not contacted with the test agent. Measuring procedures of HIF1α activity or the expression level of an HIF1α inducible factor in the control group is as described above.

The test agent is determined to have a regulatory effect on the activity of HIF1α or an HIF1α inducible factor if the activity of HIF1α or the HIF1α inducible factor in the test group is increased or decreased as compared to the activity of HIF1α or the HIF1α inducible factor in the control group based on the results of comparison.

For example, the test agent is determined to have a decreasing effect on the activity of HIF1α or the HIF1α inducible factor if the activity of HIF1α or the HIF1α inducible factor in the test group is decreased as compared to the activity of HIF1α or the HIF1α inducible factor in the control group. In a preferred embodiment, the test agent is determined to have a decreasing effect on the activity of HIF1α or the HIF1α inducible factor if the activity of HIF1α or the HIF1α inducible factor in the test group is statistically significantly decreased as compared to the activity of HIF1α or the HIF1α inducible factor in the control group. In another preferred embodiment, the test agent is determined to have a decreasing effect on the activity of HIF1α or the HIF1α inducible factor, if the activity of HIF1α or the HIF1α inducible factor in the test group is 95% or less, preferably 90% or less, more preferably 85% or less, and further preferably 80% or less when the activity of HIF1α or the HIF1α inducible factor in the control group is taken as 100%.

On the other hand, the test agent is determined to have an increasing effect on the activity of HIF1α or the HIF1α inducible factor if the activity of HIF1α or the HIF1α inducible factor in the test group is increased as compared to the activity of HIF1α or the HIF1α inducible factor in the control group. In a preferred embodiment, the test agent is determined to have an increasing effect on the activity of HIF1α or the HIF1α inducible factor if the activity of HIF1α or the HIF1α inducible factor in the test group is statistically significantly increased as compared to the activity of HIF1α or the HIF1α inducible factor in the control group. In another preferred embodiment, the test agent is determined to have an increasing effect on the activity of HIF1α or the HIF1α inducible factor, if the activity of HIF1α or the HIF1α inducible factor in the test group is 105% or more, preferably 110% or more, more preferably 115% or more, and further preferably 120% or more when the activity of HIF1α or the HIF1α inducible factor in the control group is taken as 100%.

The test agent which is determined to have a regulatory effect on the activity of HIF1α or the HIF1α inducible factor based on the results of determination is selected as an androgen receptor activity regulator selective to sebaceous glands or hair follicles. More specifically, the test agent which is determined to have a decreasing effect on the activity of HIF1α or the HIF1α inducible factor is selected as an androgen receptor activity repressor selective to sebaceous glands or hair follicles, a sebum secretion repressor, an agent for preventing or improving acne, an agent for preventing or improving alopecia, or an agent for preventing or improving hypertrichosis. On the other hand, the test agent which is determined to have an increasing effect on the activity of HIF1α or the HIF1α inducible factor is selected as an androgen receptor activator selective to sebaceous glands or hair follicles, a sebum secretion activator, or an agent for preventing or improving senile xerosis.

Examples of agents which have a decreasing effect on the activity of HIF1α or an HIF1α inducible factor include siRNAs for the gene of HIF1α or the HIF1α inducible factor. Therefore, the siRNAs for the gene of HIF1α or an HIF1α inducible factor can be used to repress an androgen receptor activity in a sebaceous gland- or hair follicle-selective manner, to repress sebum secretion, to prevent or improve acne, to prevent or improve alopecia, or to prevent or improve hypertrichosis.

The use of the siRNAs in the present disclosure may be therapeutic use or non-therapeutic use. Examples of the therapeutic use of the present disclosure include use of the siRNAs in human and non-human mammals suffering from alopecia or hypertrichosis. Examples of the non-therapeutic use include use for repressing sebum secretion, use for improving oily skin, or use for preventing acne for cosmetic purposes and use for preventing head hair loss or decrease in body hair for cosmetic purposes.

Accordingly, still another embodiment as described herein is an androgen receptor activity repressor selective to sebaceous glands or hair follicles, a sebum secretion repressor, an agent for preventing or improving acne, an agent for preventing or improving alopecia, or an agent for preventing or improving hypertrichosis, comprising, as an active ingredient, at least one selected from the group consisting of siRNAs for each of HIF1α gene, ENO1 gene, LDHA gene, PGK1 gene, GPI gene, and HK1 gene.

Still another embodiment of the present disclosure is a method of repressing an androgen receptor activity in a sebaceous gland- or hair follicle-selective manner, a method of repressing sebum secretion, a method of preventing or improving acne, a method of preventing or improving alopecia, or a method of preventing or improving hypertrichosis, the method comprising administering at least one selected from the group consisting of siRNAs for each of HIF1α gene, ENO1 gene, LDHA gene, PGK1 gene, GPI gene, and HK1 gene to sebaceous gland cells or hair follicle cells in a subject. Subjects in the methods of the disclosure include human and non-human mammals. Preferably, the subjects include a subject who needs or desires repression of activity of androgen receptors in a sebaceous gland- or hair follicle-selective manner. More specifically, the subjects include a subject who needs or desires repression of sebum secretion, prevention or improvement of acne, prevention or improvement of alopecia, or prevention or improvement of hypertrichosis.

As disclosed herein, the siRNAs are preferably administered to sebaceous gland cells in the method of repressing sebum secretion and the method of preventing or improving acne. As disclosed herein, the siRNAs are preferably administered to hair follicle cells in the head in the method of preventing or improving alopecia. As disclosed herein, the siRNAs are preferably administered to hair follicle cells in the head or in any site other than head (such as face, neck, arm, hand, leg, foot, and trunk) in the method of preventing or improving hypertrichosis.

In a preferred embodiment of the present disclosure, the siRNAs are transdermally administered or topically applied onto the skin.

In a preferred embodiment of the present disclosure, the non-human mammals include mouse, rat, and other non-human mammals.

The siRNAs for the gene of HIF1α or an HIF1α inducible factor can be designed or synthesized by using any known software (such as siDirect) or custom synthesis service (provided by Life technologies, Sigma-Aldrich, and other manufacturers). Generally, an siRNA is designed to form a double-stranded RNA (or RNA/DNA) as short as 21 to 30 bases or preferably 21 to 25 bases. An siRNA may have blunt ends and typically has a protruding part (overhang) of about two bases at each 3'-end of the double strand. The protruding part is often designed to have, but not limited to, TT.

Preferable examples of siRNAs which can be used herein include a double-stranded RNA or an RNA/DNA duplex consisting of RNA having any of the following sequences and a complementary strand thereof. These siRNAs are directed to HIF1α gene.
5'-CCAGCCGCUGGAGACACAAUCAUAU-3' (SEQ ID NO: 8)
5'-GGGAUUAACUCAGUUUGAACUAACU-3' (SEQ ID NO: 9)
5'-GAAAUUCCUUUAGAUAGCAAGACUU-3' (SEQ ID NO: 10)
The RNA as set forth in SEQ ID NO: 8 corresponds to the bases at positions 1204 to 1228 in the SEQ ID NO: 1. The RNA as set forth in SEQ ID NO: 9 corresponds to the bases at positions 360 to 384 in the SEQ ID NO: 1. The RNA as set forth in SEQ ID NO: 10 corresponds to the bases at positions 700 to 724 in the SEQ ID NO: 1.

Further examples of siRNAs which can be used herein include a double stranded RNA or an RNA/DNA duplex consisting of an RNA which specifically binds the bases at positions 1204 to 1228 in the SEQ ID NO: 1, the bases at positions 360 to 384 in the SEQ ID NO: 1, or the bases at positions 700 to 724 in the SEQ ID NO: 1 and a complementary strand of the RNA. Preferable examples of the siRNAs include a double stranded RNA or an RNA/DNA duplex consisting of an RNA having 90% or more and preferably 95% or more sequence identity to the RNA as set forth in either SEQ ID NO: 8, SEQ ID NO: 9, or SEQ ID NO: 10 and a complementary strand of the RNA.

Further agents, manufacturing methods, applications, or methods are disclosed herein below as exemplary embodiments of the present disclosure.
[1] A method of evaluating or selecting an androgen receptor activity regulator selective to sebaceous glands or hair follicles, the method comprising:
   (A) applying a test agent to cells from sebaceous glands or hair follicles under a hypoxic condition;
   (B) measuring HIF1α activity in the cells;
   (C) comparing the HIF1α activity measured in the (B) with HIF1α activity in a control group; and
   (D) determining a regulatory effect of the test agent on the HIF1α activity based on the result in the (C).
[2] The method according to [1], preferably further comprising:
   (E) selecting the test agent which is determined to have a decreasing effect on the HIF1α activity in the (D) as an androgen receptor activity repressor selective to sebaceous glands or hair follicles, a sebum secretion repressor, an agent for preventing or improving acne, an agent for preventing or improving alopecia, or an agent for preventing or improving hypertrichosis.
[3] The method according to [1], preferably further comprising:
   (E) selecting the test agent which is determined to have an increasing effect on the HIF1α activity in the (D) as an androgen receptor activator selective to sebaceous glands or hair follicles, a sebum secretion activator, or an agent for preventing or improving senile xerosis.
[4] A method of evaluating or selecting a sebum secretion regulator, an agent for preventing or improving acne, an agent for preventing or improving alopecia, an agent for preventing or improving hypertrichosis, or an agent for preventing or improving senile xerosis, the method comprising:
   (A) applying a test agent to cells from sebaceous glands or hair follicles under a hypoxic condition;
   (B) measuring HIF1α activity in the cells;
   (C) comparing the HIF1α activity measured in the (B) with HIF1α activity in a control group; and
   (D) determining a regulatory effect of the test agent on the HIF1α activity based on the result in the (C).
[5] The method according to [4], preferably further comprising:
   (E) selecting the test agent which is determined to have a decreasing effect on the HIF1α activity in the (D) as a sebum secretion repressor, an agent for preventing or improving acne, an agent for preventing or improving alopecia, or an agent for preventing or improving hypertrichosis.
[6] The method according to [4], preferably further comprising:
   (E) selecting the test agent which is determined to have an increasing effect on the HIF1α activity in the (D) as a sebum secretion activator or an agent for preventing or improving senile xerosis.
[7] The method according to any one of [1] to [6], preferably wherein the cells from sebaceous glands or hair follicles are from human.
[8] The method according to any one of [1] to [6], preferably wherein the cells from sebaceous glands are cells of an established human sebaceous gland cell line.
[9] The method according to any one of [1] to [8], preferably wherein the hypoxic condition is a condition of a concentration of oxygen in a culture atmosphere of about 5% or less and more preferably from about 0.1% to about 5%.
[10] The method according to any one of [1] to [9], preferably wherein the measuring the HIF1α activity is measuring a binding level of HIF1α to hypoxia response element.
[11] The method according to any one of [1] to [9], preferably wherein the measuring HIF1α activity is measuring an expression level of HIF1α gene or protein.
[12] The method according to [11], preferably wherein the HIF1α gene is a polynucleotide consisting of the nucleotide sequence as set forth in SEQ ID NO: 1 or a nucleotide sequence having at least 90% identity to the nucleotide sequence as set forth in SEQ ID NO: 1.
[13] The method according to any one of [1] to [12], preferably wherein the HIF1α is a protein consisting of the amino acid sequence as set forth in SEQ ID NO: 2 or an amino acid sequence having at least 90% identity to the amino acid sequence as set forth in SEQ ID NO: 2.
[14] The method according to any one of [1] to [13], preferably wherein the test agent is determined to have a decreasing effect on the HIF1α activity if the activity measured in the (B) is statistically significantly decreased as compared to the activity in the control group.
[15] The method according to any one of [1] to [13], preferably wherein the test agent is determined to have an increasing effect on the HIF1α activity if the activity measured in the (B) is statistically significantly increased as compared to the activity in the control group.
[16] The method according to any one of [1] to [13], preferably wherein the test agent is determined to have a decreasing effect on the HIF1α activity if the activity measured in the (B) is 95% or less, preferably 90% or less, more preferably 85% or less, and further preferably 80% or less when the HIF1α activity in the control group is taken as 100%.
[17] The method according to any one of [1] to [13], preferably wherein the test agent is determined to have an increasing effect on the HIF1α activity if the activity measured in the (B) is 105% or more, preferably 110% or more, more preferably 115% or more, and further preferably 120% or more when the HIF1α activity in the control group is taken as 100%.
[18] A method of evaluating or selecting an androgen receptor activity regulator selective to sebaceous glands or hair follicles, the method comprising:
   (A) applying a test agent to cells from sebaceous glands or hair follicles under a hypoxic condition;
   (B') measuring activity of a factor induced by HIF1α in the cells;
   (C') comparing the activity measured in the (B') with activity of the factor in a control group; and
   (D') determining a regulatory effect of the test agent on the activity of the factor based on the result in the (C').
[19] The method according to [18], preferably further comprising:
   (E') selecting the test agent which is determined to have a decreasing effect on the activity of the factor induced by HIF1α in the (D') as an androgen receptor activity repressor selective to sebaceous glands or hair follicles, a sebum secretion repressor, an agent for preventing or improving acne, an agent for preventing or improving alopecia, or an agent for preventing or improving hypertrichosis.
[20] The method according to [18], preferably further comprising:
   (E') selecting the test agent which is determined to have an increasing effect on the activity of the factor induced by HIF1α in the (D') as an androgen receptor activator selective to sebaceous glands or hair follicles, a sebum secretion activator, or an agent for preventing or improving senile xerosis.
[21] A method of evaluating or selecting a sebum secretion regulator, an agent for preventing or improving acne, an agent for preventing or improving alopecia, an agent for preventing or improving hypertrichosis, or an agent for preventing or improving senile xerosis, the method comprising:
   (A) applying a test agent to cells from sebaceous glands or hair follicles under a hypoxic condition;
   (B') measuring activity of a factor induced by HIF1α in the cells;
   (C') comparing the activity measured in the (B') with activity of the factor in a control group; and
   (D') determining a regulatory effect of the test agent on the activity of the factor based on the result in the (C').
[22] The method according to [21], preferably further comprising:
   (E') selecting the test agent which is determined to have a decreasing effect on the activity of the factor induced by HIF1α in the (D') as a sebum secretion repressor, an agent for preventing or improving acne, an agent for preventing or improving alopecia, or an agent for preventing or improving hypertrichosis.
[23] The method according to [21], preferably further comprising:
   (E') selecting the test agent which is determined to have an increasing effect on the activity of the factor induced by HIF1α in the (D') as a sebum secretion activator or an agent for preventing or improving senile xerosis.
[24] The method according to any one of [18] to [23], preferably wherein the cells from sebaceous glands or hair follicles are from human.
[25] The method according to any one of [18] to [23], preferably wherein the cells from sebaceous glands are cells of an established human sebaceous gland cell line.
[26] The method according to any one of [18] to [25], wherein the hypoxic condition is a condition preferably of a concentration of oxygen in a culture atmosphere of about 5% or less and more preferably from about 0.1% to about 5%.
[27] The method according to any one of [18] to [26], wherein the measuring the activity of the factor induced by HIF1α is preferably measuring an expression level of at least one gene selected from the group consisting of ENOl, LDHA, PGK1, GPI, and HK1, or an expression level of at least one enzyme selected from the group consisting of enzymes coded by the genes, or a level of enzymatic activity of at least one enzyme selected from the group consisting of enzymes coded by the genes.
[28] The method according to [27], preferably
   wherein the ENOl gene is a polynucleotide consisting of the nucleotide sequence as set forth in SEQ ID NO: 3 or a nucleotide sequence having at least 90% identity to the nucleotide sequence as set forth in SEQ ID NO: 3, wherein the LDHA gene is a polynucleotide consisting of the nucleotide sequence as set forth in SEQ ID NO: 4 or a nucleotide sequence having at least 90% identity to the nucleotide sequence as set forth in SEQ ID NO: 4, wherein the PGK1 gene is a polynucleotide consisting of the nucleotide sequence as set forth in SEQ ID NO: 5 or a nucleotide sequence having at least 90% identity to the nucleotide sequence as set forth in SEQ ID NO: 5, wherein the GPI gene is a polynucleotide consisting of the nucleotide sequence as set forth in SEQ ID NO: 6 or a nucleotide sequence having at least 90% identity to the nucleotide sequence as set forth in SEQ ID NO: 6, and wherein the HK1 gene is a polynucleotide consisting of the nucleotide sequence as set forth in SEQ ID NO: 7 or a nucleotide sequence having at least 90% identity to the nucleotide sequence as set forth in SEQ ID NO: 7.
[29] The method according to any one of [18] to [28], preferably wherein the test agent is determined to have a decreasing effect on the activity of the factor induced by HIF1α if the activity measured in the (B') is statistically significantly decreased as compared to the activity in the control group.
[30] The method according to any one of [18] to [28], preferably wherein the test agent is determined to have an increasing effect on the activity of the factor induced by HIF1α if the activity measured in the (B') is statistically significantly increased as compared to the activity in the control group.
[31] The method according to any one of [18] to [28], preferably wherein the test agent is determined to have a decreasing effect on the activity of the factor induced by HIF1α, if the activity measured in the (B') is 95% or less, preferably 90% or less, more preferably 85% or less, and further preferably 80% or less when the HIF1α activity in the control group is taken as 100%.
[32] The method according to any one of [18] to [28], preferably wherein the test agent is determined to have an increasing effect on the activity of the factor induced by HIF1α, if the activity measured in the (B') is 105% or more, preferably 110% or more, more preferably 115% or more, and further preferably 120% or more when the HIF1α activity in the control group is taken as 100%.
[33] An androgen receptor activity repressor selective to sebaceous glands or hair follicles comprising, as an active ingredient, at least one selected from the group consisting of siRNAs for each of HIF1α gene, ENOl gene, LDHA gene, PGK1 gene, GPI gene, and HK1 gene.
[34] A sebum secretion repressor comprising, as an active ingredient, at least one selected from the group consisting of siRNAs for each of HIF1α gene, ENOl gene, LDHA gene, PGK1 gene, GPI gene, and HK1 gene.
[35] An agent for preventing or improving acne comprising, as an active ingredient, at least one selected from the group consisting of siRNAs for each of HIF1α gene, ENOl gene, LDHA gene, PGK1 gene, GPI gene, and HK1 gene.
[36] An agent for preventing or improving alopecia comprising, as an active ingredient, at least one selected from the group consisting of siRNAs for each of HIF1α gene, ENO1 gene, LDHA gene, PGK1 gene, GPI gene, and HK1 gene.
[37] An agent for preventing or improving hypertrichosis comprising, as an active ingredient, at least one selected from the group consisting of siRNAs for each of HIF1α gene, ENO1 gene, LDHA gene, PGK1 gene, GPI gene, and HK1 gene.
[38] Use of at least one selected from the group consisting of siRNAs for each of HIF1α gene, ENOl gene, LDHA gene, PGK1 gene, GPI gene, and HK1 gene for the manufacture of an androgen receptor activity repressor selective to sebaceous glands or hair follicles.
[39] Use of at least one selected from the group consisting of siRNAs for each of HIF1α gene, ENOl gene, LDHA gene, PGK1 gene, GPI gene, and HK1 gene for the manufacture of a sebum secretion repressor.
[40] Use of at least one selected from the group consisting of siRNAs for each of HIF1α gene, ENOl gene, LDHA gene, PGK1 gene, GPI gene, and HK1 gene for the manufacture of an agent for preventing or improving acne.
[41] Use of at least one selected from the group consisting of siRNAs for each of HIF1α gene, ENOl gene, LDHA gene, PGK1 gene, GPI gene, and HK1 gene for the manufacture of an agent for preventing or improving alopecia.
[42] Use of at least one selected from the group consisting of siRNAs for each of HIF1α gene, ENOl gene, LDHA gene, PGK1 gene, GPI gene, and HK1 gene for the manufacture of an agent for preventing or improving hypertrichosis.
[43] Use of at least one selected from the group consisting of siRNAs for each of HIF1α gene, ENOl gene, LDHA gene, PGK1 gene, GPI gene, and HK1 gene for repressing an androgen receptor activity in a sebaceous gland- or hair follicle-selective manner.
[44] Use of at least one selected from the group consisting of siRNAs for each of HIF1α gene, ENOl gene, LDHA gene, PGK1 gene, GPI gene, and HK1 gene for repressing sebum secretion.
[45] Use of at least one selected from the group consisting of siRNAs for each of HIF1α gene, ENOl gene, LDHA gene, PGK1 gene, GPI gene, and HK1 gene for preventing or improving acne.
[46] Use of at least one selected from the group consisting of siRNAs for each of HIF1α gene, ENOl gene, LDHA gene, PGK1 gene, GPI gene, and HK1 gene for preventing or improving alopecia.
[47] Use of at least one selected from the group consisting of siRNAs for each of HIF1α gene, ENO1 gene, LDHA gene, PGK1 gene, GPI gene, and HK1 gene for preventing or improving hypertrichosis.
[48] In any one of [43] to [47], preferably the use is non-therapeutic use.
[49] At least one selected from the group consisting of siRNAs for each of HIF1α gene, ENO1 gene, LDHA gene, PGK1 gene, GPI gene, and HK1 gene for use in the repression of an androgen receptor activity in a sebaceous gland- or hair follicle-selective manner.
[50] At least one selected from the group consisting of siRNAs for each of HIF1α gene, ENO1 gene, LDHA gene, PGK1 gene, GPI gene, and HK1 gene for use in the repression of sebum secretion.
[51] At least one selected from the group consisting of siRNAs for each of HIF1α gene, ENO1 gene, LDHA gene, PGK1 gene, GPI gene, and HK1 gene for use in the prevention or improvement of acne.
[52] At least one selected from the group consisting of siRNAs for each of HIF1α gene, ENO1 gene, LDHA gene, PGK1 gene, GPI gene, and HK1 gene for use in the prevention or improvement of alopecia.
[53] At least one selected from the group consisting of siRNAs for each of HIF1α gene, ENO1 gene, LDHA gene, PGK1 gene, GPI gene, and HK1 gene for use in the prevention or improvement of hypertrichosis.
[54] A method of repressing an androgen receptor activity in a sebaceous gland- or hair follicle-selective manner, the method comprising administering at least one selected from the group consisting of siRNAs for each of HIF1α gene, ENO1 gene, LDHA gene, PGK1 gene, GPI gene, and HK1 gene to sebaceous gland cells or hair follicle cells in a subject.
[55] A method of repressing sebum secretion, the method comprising administering at least one selected from the group consisting of siRNAs for each of HIF1α gene, ENO1 gene, LDHA gene, PGK1 gene, GPI gene, and HK1 gene to sebaceous gland cells in a subject.
[56] A method of preventing or improving acne, the method comprising administering at least one selected from the group consisting of siRNAs for each of HIF1α gene, ENO1 gene, LDHA gene, PGK1 gene, GPI gene, and HK1 gene to sebaceous gland cells in a subject.
[57] A method of preventing or improving alopecia, the method comprising administering at least one selected from the group consisting of siRNAs for each of HIF1α gene, ENO1 gene, LDHA gene, PGK1 gene, GPI gene, and HK1 gene to hair follicle cells in the head of a subject.
[58] A method of preventing or improving hypertrichosis, the method comprising administering at least one selected from the group consisting of siRNAs for each of HIF1α gene, ENO1 gene, LDHA gene, PGK1 gene, GPI gene, and HK1 gene to hair follicle cells in a subject.
[59] The method according to any one of [54] to [58], preferably wherein the subject is human or a non-human mammal.
[60] The method according to any one of [54] to [59], preferably wherein the administering is transdermal administration or topical administration onto the skin.
[61] In any one of [33] to [60], preferably,
   the HIF1α gene is a polynucleotide consisting of the nucleotide sequence as set forth in SEQ ID NO: 1 or a nucleotide sequence having at least 90% identity to the nucleotide sequence as set forth in SEQ ID NO: 1,
   the ENOl gene is a polynucleotide consisting of the nucleotide sequence as set forth in SEQ ID NO: 3 or a nucleotide sequence having at least 90% identity to the nucleotide sequence as set forth in SEQ ID NO: 3,
   the LDHA gene is a polynucleotide consisting of the nucleotide sequence as set forth in SEQ ID NO: 4 or a nucleotide sequence having at least 90% identity to the nucleotide sequence as set forth in SEQ ID NO: 4,
   the PGK1 gene is a polynucleotide consisting of the nucleotide sequence as set forth in SEQ ID NO: 5 or a nucleotide sequence having at least 90% identity to the nucleotide sequence as set forth in SEQ ID NO: 5,
   the GPI gene is a polynucleotide consisting of the nucleotide sequence as set forth in SEQ ID NO: 6 or a nucleotide sequence having at least 90% identity to the nucleotide sequence as set forth in SEQ ID NO: 6, and the HK1 gene is a polynucleotide consisting of the nucleotide sequence as set forth in SEQ ID NO: 7 or a nucleotide sequence having at least 90% identity to the nucleotide sequence as set forth in SEQ ID NO: 7.
[62] In any one of [33] to [61], preferably the siRNA is a double stranded RNA or an RNA/DNA duplex consisting of RNA as set forth in SEQ ID NO: 8, SEQ ID NO: 9, or SEQ ID NO: 10 and a complementary strand thereof or is a double stranded RNA or an RNA/DNA duplex consisting of RNA having 90% or more and preferably 95% or more sequence identity to any of RNA as set forth in SEQ ID NO: 8, SEQ ID NO: 9, or SEQ ID NO: 10 and a complementary strand thereof.

### [Examples]

The present disclosure will be now described more specifically by illustrating the Examples below.

### Methods

### Reference Example 1. Cells

A human sebaceous gland cell line (SZ95) was cultured in Sebomed™ basal medium (Biochrom) supplemented with 10% FBS and 5 ng/mL Epidermal Growth Factor. A human prostate cancer cell line (LNCaP) was cultured in RPMI1640 (Life technologies) supplemented with 10% FBS. Culturing at a normal oxygen concentration was performed in the air (at the oxygen concentration of 20.9%). Culturing under a hypoxic condition was performed using AnaeroPack-Anaero 5% (MITSUBISHI GAS CHEMICAL COMPANY, INC.) and Rectangular Jar (SUGIYAMA-GEN CO., LTD) under the condition at 0.1% of oxygen concentration and 5% CO₂ and at 37°C. SZ95 was donated by Prof. Dr. Prof. h. c. Dr. h. c. Christos C. Zouboulis. LNCaP was purchased from ATCC.

### Reference Example 2. Generation of plasmid DNA

### (1) Androgen Receptor Gene

The open reading flame (ORF) region of the gene encoding human androgen receptor (AR) was amplified by PCR using PrimeSTAR® GXL DNA Polymerase (Takara Bio) and the amplified product was then inserted into pcDNA3.1 (Life technologies) digested with EcoRI using In-Fusion HD Cloning Kit (Takara Bio). The plasmid DNA of interest was purified from Escherichia coli transformed with the plasmid DNA using EndoFree Plasmid Purification Kit (Qiagen). The purified plasmid DNA was designated as pcDNA3.1-AR.

### (2) Genes of Glycolytic Enzymes

The open reading flame (ORF) regions of genes ENOl, LDHA, GAPDH, PGK1, ALDOA, HK1, GPI, and TPI encoding respectively enolase 1, lactate dehydrogenase A, glyceraldehyde-3-phosphate dehydrogenase, phosphoglycerate kinase 1, aldolase A, hexokinase 1, glucose-6-phosphate isomerase, and triosephosphate isomerase, which are enzymes involved in glycolysis, were inserted into pcDNA3.1 (Life technologies) by the same procedure as in the (1). The plasmid DNA of interest was purified from Escherichia coli transformed with the plasmid DNA using EndoFree Plasmid Purification Kit (Qiagen).

### (3) Marker gene of HIF1α activity

pGL4.42 (Promega), which is a plasmid DNA for determining HIF1α activity, can express firefly luciferase under the control of HRE. Cytomegalovirus enhance/early promoter and hygromycin resistance gene which are downstream of the firefly luciferase gene in the pGL4.42 were digested with Sal1 and BamH1. Renilla luciferase gene linked to SV40 promoter was then inserted into the Sal1 and BamH1 sites using In-Fusion HD Cloning Kit (Takara Bio). The plasmid DNA of interest was purified from Escherichia coli transformed with the plasmid DNA using EndoFree Plasmid Purification Kit (Qiagen). The purified plasmid DNA was designated as pGL4.42 (Rluc).

### Reference Example 3. RNA extraction, cDNA synthesis, and real time PCR

Total RNA was extracted from cells using RNeasy® Mini kit according to the accompanying protocol. cDNA was synthesized from the extracted total RNA using QuantiTect® reverse transcription kit (QIAGEN). The mRNA expression level of each gene was quantified by real time PCR using TaqMan® Universal master Mix (Life technologies) and TaqMan® probe (Life technologies). The quantified expression level was normalized with the expression level of RPLP0.

### Reference Example 4. HIF1α Immunohistochemical Staining

Tissue samples for immunohistochemical staining were cut into frozen sections and then fixed in acetone cooled at -20°C for 10 minutes. Subsequently, the sections were stained using Histofine kit (NICHIREI BIOSCIENCE INC.) according to the accompanying protocol. Anti-HIF1α antibody (Novus Biologicals) was diluted at 1/300 for use in the staining. DAB solution (1mM DAB, 50mM Tris-HCL buffer (pH 7.6), and 0.006% hydrogen peroxide) was used for color development while hematoxylin solution was used for counter staining. To semiquantitatively analyze the intensity of expression of HIF1α in the tissue samples, HIF1α-positive cells (which were stained in brown) and HIF1α-negative cells (which were stained in purple) were counted under a microscope. Cells which were determined whether they were HIF1α positive or negative included AR positive cells in sebaceous gland, hair follicle, ovary, and testis, wherein the AR positive cells were cells composing sebaceous glands, cells composing hair follicles, ovarian follicle cells in ovary, and cells composing seminiferous tubules in testis.

### Example 1. HIF1α expression in sebaceous glands

Samples of sebaceous gland and hair follicle, testis tissue, and ovary tissue were prepared from a man aged 35, a man aged 33, and a woman aged 30, respectively. Each of the tissue samples was immunohistochemically stained against HIF1α. The results revealed that HIF1α expression, which is HIF1α positive rate, in sebaceous gland and hair follicle was markedly higher than the HIF1α expression in testis and ovary (Table 1).

**[Table 1]**

| | Sebaceous gland | Hair follicle | Testis | Ovary |
|---|---|---|---|---|
| Total number of cells determined | 300 cells | 300 cells | 300 cells | 300 cells |
| Number of positive cells | 157 cells | 107 cells | 4 cells | 5 cells |
| Number of negative cells | 143 cells | 293 cells | 296 cells | 295 cells |
| Positive rate | 47.60% | 35.60% | 1.30% | 1.60% |

### Example 2. Effect of expression of glycolytic enzymes on the AR activity

Cells over-expressing AR were generated according to the procedure described in J Steroid Biochem & Mol Biol, 123: 58-64, 2011. Effect of expression of glycolytic enzymes on the AR activity was examined.

SZ95 cells were seeded in a 96-well plate and the cell medium was changed to Sebomed™ basal medium (Biochrom) supplemented with 5% charcoal-treated serum. A drug resistance gene Hygro was removed using restriction enzymes from pGL4.36 (Promega) having the AR binding sequence (MMTV) upstream of the firefly luciferase gene. Renilla luciferase gene was cloned into the restriction sites. The modified pGL4.36, the pcDNA3.1-AR generated in the Reference Example 2(1), and the plasmid DNA of pcDNA3.1 incorporating ORF of any of various glycolytic enzymes generated in the Reference Example 2(2) were transferred into the SZ95 cells using ViaFect (Promega) according to the protocol supplied with the product. As a control, the plasmid DNA which has no sequence at the multiple cloning site in pcDNA3.1 was transferred. At 24 hours after transfer, the cell medium was changed to the medium containing dihydrotestosterone (DHT; final concentration of 1 nM). After culturing for 6 hours, the AR activity in the cells was determined. In the determination of AR activity, the luminescence intensity of firefly luciferase (which was an indicator of transcriptional activation of AR mediated by MMTV) and Renilla luciferase (which was an indicator of plasmid transfer efficiency) in the cells was determined using Dual-Glo Luciferase Assay System (Promega) according to the accompanying protocol. Relative Light Unit (RLU) was calculated by dividing the luminescence intensity of firefly luciferase by the luminescence intensity of Renilla luciferase. The relative RLU value compared to the RLU value of the control was obtained as the AR activity in the cells.

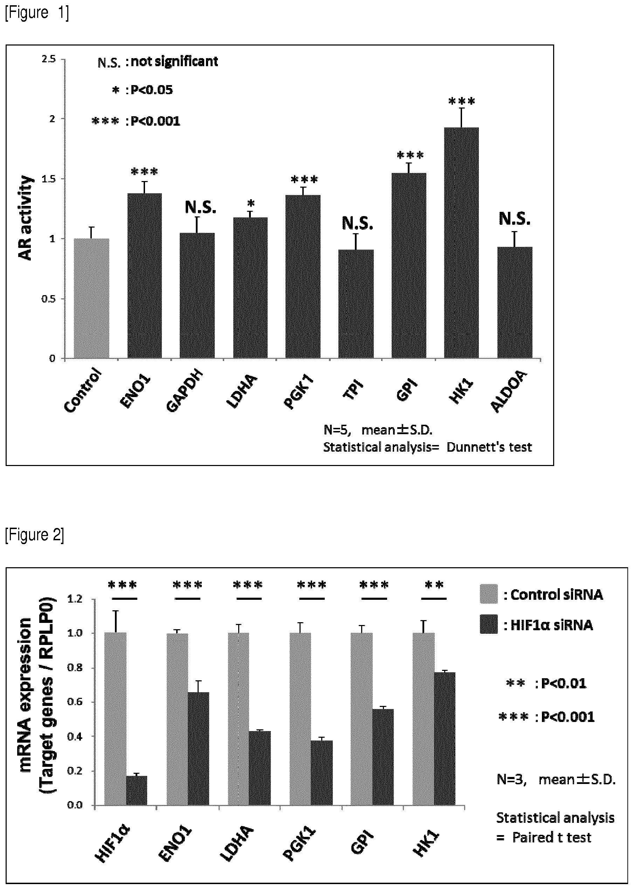

The results are shown in FIG. 1 and Table 2. The AR activities in the cells over-expressing ENO1, LDHA, PGK1, GPI, or HK1 were significantly higher than the AR activity in the cells transformed with the control plasmid. It has not been reported so far that glycolytic enzymes can regulate AR activity. It is now revealed for the first time that some glycolytic enzymes can regulate AR activity in sebaceous gland cells.

**[Table 2]**

| | AR activity (RLU) | | |
|---|---|---|---|
| | AVE | SD | Dunnett's test (compared with control, n=5) |
| Control | 1.0000 | 0.0972 | - |
| ENO1 | 1.3752 | 0.1051 | p < 0.001 |
| GAPDH | 1.0515 | 0.1309 | N.S. |
| LDHA | 1.1745 | 0.0529 | p < 0.05 |
| PGK1 | 1.3604 | 0.0693 | p < 0.001 |
| TPI | 0.9073 | 0.1330 | N.S. |
| GPI | 1.5478 | 0.0866 | p < 0.001 |
| HK1 | 1.9274 | 0.1629 | p < 0.001 |
| ALDOA | 0.9291 | 0.1317 | N.S. |

### Example 3. Effect of HIF1α on the expression of glycolytic enzymes

The present inventors investigated the possibility of regulating the expression of glycolytic enzymes via HIF1α in sebaceous gland cells.

SZ95 cells were seeded in a 96-well plate and the cell medium was changed to a medium supplemented with 5% charcoal-treated serum. Control siRNAs (Stealth RNAi™ shiRNA Negative Controls, 12935-400 and 12935-200, Life technologies) or siRNAs for HIF1α (Stealth Select RNAi™ shiRNA, HSS104774, HSS104775, and HSS179231, Life technologies) were transferred to the cells using Lipofectamine™ RNAiMAX Transfection Reagent (Life technologies) according to the protocol supplied with the product. The cells were cultured for 24 hours under a hypoxic condition (at about 0.1% of oxygen concentration). RNA was collected from the cells according to the procedure described in the Reference Example 3. Expression of ENOl, LDHA, PGK1, GPI, and HK1 gene, which increased AR activity in the Example 2, was quantified by real time PCR.

The results are shown in FIG. 2 and Table 3. The expression of ENOl, LDHA, PGK1, GPI, and HK1 was significantly decreased by repressing the expression of HIF1α.

**[Table 3]**

| | mRNA expression | | | | |
|---|---|---|---|---|---|
| | Control siRNA | | HIF1α siRNA | | *t-*test (n=3) |
| | AVE | SD | AVE | SD | |
| HIF1α | 1.0052 | 0.1249 | 0.1685 | 0.0175 | p < 0.001 |
| ENO1 | 1.0002 | 0.0228 | 0.6554 | 0.0690 | p < 0.001 |
| LDHA | 1.0009 | 0.0511 | 0.4315 | 0.0080 | p < 0.001 |
| PGK | 1.0012 | 0.0589 | 0.3786 | 0.0163 | p < 0.001 |
| GPI | 1.0006 | 0.0441 | 0.5607 | 0.0130 | p < 0.001 |
| HK1 | 1.0017 | 0.0711 | 0.7703 | 0.0139 | p < 0.01 |

### Example 4. Effect of HIF1α on the AR activity in sebaceous gland cells

SZ95 cells were seeded in a 96-well plate and the cell medium was changed to a medium supplemented with 5% charcoal-treated serum. Control siRNAs or siRNAs for HIF1α (Life technologies) were transferred to the cells using Lipofectamine™ RNAiMAX Transfection Reagent (Life technologies) according to the protocol supplied with the product. After culturing the cells for 24 hours, the cells were transformed with the pcDNA3.1-AR generated in the Reference Example 2(1) and the modified pGL4.36, which is the same as used in the Example 2 and has the cloned Renilla luciferase gene, using ViaFect (Promega) according to the protocol supplied with the product. After 24 hours, the cell medium was changed to a medium containing DHT (at the final concentration of 0.1, 1, or 10 nM) and the cells were cultured under a hypoxic condition (at 0.1% of oxygen concentration). As a control, the cell medium was changed to a medium containing 100% ethanol and the cells were cultured under a hypoxic condition (at 0.1% of oxygen concentration). After 24 hours, the Relative Light Unit (RLU) was calculated by the same procedure as in the Example 2 and obtained as the AR activity in the cells.

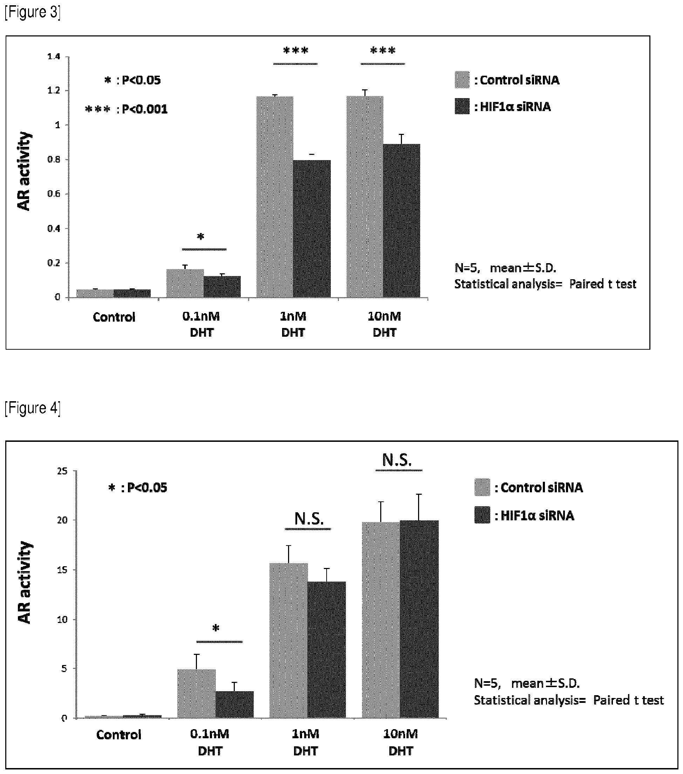

The results are shown in FIG. 3 and Table 4. The AR activities were significantly decreased at all DHT concentrations ranging from 0.1 to 10 nM in the cells in which the expression of HIF1α was repressed.

**[Table 4]**

| siRNA | DHT | AR activity (RLU) | | |
|---|---|---|---|---|
| | | AVE | SD | *t-*test (compared with control, n=5) |
| Control | Cont. (EtOH only) | 0.0431 | 0.0069 | |
| | 0.1 nM | 0.1628 | 0.0247 | |
| | 1 nM | 1.1650 | 0.0134 | |
| | 10 nM | 1.1679 | 0.0336 | |
| HIF1 | Cont. (EtOH only) | 0.0428 | 0.0078 | N.S. |
| | 0.1 nM | 0.1244 | 0.0109 | p < 0.05 |
| | 1 nM | 0.7962 | 0.0372 | p < 0.001 |
| | 10 nM | 0.8899 | 0.0550 | p < 0.001 |

### Comparative Example 1. Effect of HIF1α on the AR activity in prostate cancer cells

It was reported that HIF1α can regulate AR activity in the low concentration range of DHT equal to or less than 0.1 nM in a prostate cancer cell LNCaP (J Steroid Biochem & Mol Biol, 123: 58-64, 2011). However, DHT and testosterone in a normal body are believed to be present at a higher concentration (J Clin Endocrinol Metab, 1994, 79: 703-706; J Clin Endocrinol Metab, 1998, 83: 2266-2274). The present inventors performed the same experiments as in the Example 4 to analyze the contribution of HIF1α to the AR activity at higher DHT concentrations in LNCaP cells. As a result, as in the previous report, the AR activity was significantly decreased at the DHT concentration of 0.1 nM in LNCaP cells due to repression of HIF1α expression. However, significant alteration of the AR activity due to repression of HIF1α expression was not confirmed at the DHT concentrations ranging from 1 to 10 nM (FIG. 4 and Table 5). These results revealed that HIF1α expressed in sebaceous gland cells regulated AR activity in a wide range of DHT concentration as compared to in prostate cancer cells. On the other hand, it is indicated that HIF1α will not regulate AR activity at a normal concentration range of DHT in prostate cancer.

**[Table 5]**

| siRNA | DHT | AR activity (RLU) | | |
|---|---|---|---|---|
| | | AVE | SD | *t-*test (compared with control, n=5) |
| Control | Cont. (EtOH only) | 0.2235 | 0.0252 | |
| | 0.1nM DHT | 4.8985 | 1.5016 | |
| | 1nM DHT | 15.6709 | 1.7621 | |
| | 10nM DHT | 19.8058 | 2.0637 | |
| HIF1 | Cont. (EtOH only) | 0.2814 | 0.1297 | N.S. |
| | 0.1nM DHT | 2.6850 | 0.9035 | p < 0.05 |
| | 1nM DHT | 13.7749 | 1.3721 | N.S. |
| | 10nM DHT | 19.9923 | 2.6537 | N.S. |

### Example 5. Effect of retinoic acid on the HIF1α activity in sebaceous gland cells

The effect of retinoic acid, which is known to be a drug for improving acne (Br J Dermatol, 107: 583-590, 1982), on the HIF1α activity was examined by adding retinoic acid into sebaceous gland cells.

The plasmid DNA pGL4.42 (Rluc) generated in the Reference Example 2(3) for determining HIF1α activity was transferred into SZ95 cells. After 24 hours, retinoic acid was added into the medium and the cells were cultured under a hypoxic condition. As a control, the medium containing 100% ethanol solvent was used. After 15 hours, the Relative Light Unit (RLU) was calculated by the same procedure as in the Example 2 and considered as the HIF1α activity in the cells.

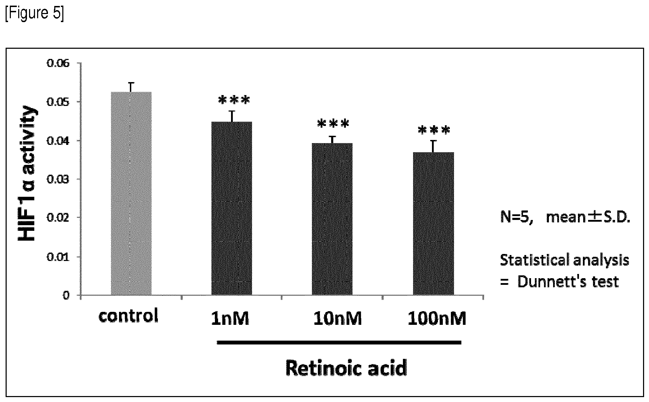

The results are shown in FIG. 5 and Table 6. HIF1α activity in the cells cultured in the medium containing retinoic acid was decreased in a concentration-dependent manner as compared to in the control cells.

**[Table 6]**

| | | HIF1 activity (RLU) | | |
|---|---|---|---|---|
| | | AVE | SD | Dunnett's test (compared with control, n=5) |
| Control | | 0.0525 | 0.0023 | - |
| Retinoic acid | 1nM | 0.0447 | 0.0028 | P<0.001 |
| | 10nM | 0.0392 | 0.0019 | P<0.001 |
| | 100nM | 0.0368 | 0.0031 | P<0.001 |

## Claims

1. An in vitro method of evaluating or selecting an androgen receptor activity regulator selective to sebaceous glands or hair follicles, the method comprising the steps:
(A) applying a test agent to cells from sebaceous glands or hair follicles under a hypoxic condition;
(B') measuring activity of a factor induced by HIF1α in the cells, wherein the factor induced by HIF1α is a glycolytic enzyme;
(C') comparing the activity measured in the (B') with activity of the factor in a control group; and
(D') determining a regulatory effect of the test agent on the activity of the factor based on the result in the (C').

2. The method according to claim 1, further comprising: (E') selecting the test agent which is determined to have a decreasing effect on the activity of the factor induced by HIF1α in the (D') as an androgen receptor activity repressor selective to sebaceous glands or hair follicles, a sebum secretion repressor, an agent for preventing or improving acne, an agent for preventing or improving alopecia, or an agent for preventing or improving hypertrichosis.

3. The method according to claim 1, further comprising: (E') selecting the test agent which is determined to have an increasing effect on the activity of the factor induced by HIF1α in the (D') as an androgen receptor activator selective to sebaceous glands or hair follicles, a sebum secretion activator, or an agent for preventing or improving senile xerosis.

4. The method according to any one of claims 1 to 3, wherein the measuring the activity of the factor induced by HIF1α is measuring an expression level of at least one gene selected from the group consisting of ENO1, LDHA, PGK1, GPI, and HK1 or an expression level or a level of enzymatic activity of at least one enzyme selected from the group consisting of enzymes coded by these genes.

5. The method according to any one of claims 1 to 4, wherein the cells from sebaceous glands or hair follicles are from human.

6. The method according to any one of claims 1 to 5, wherein the hypoxic condition is a condition of a concentration of oxygen in a culture atmosphere of 5% or less.

7. Non-therapeutic use of at least one selected from the group consisting of siRNAs for each of HIF1α gene, ENO1 gene, LDHA gene, PGK1 gene, GPI gene, and HK1 gene for use in repressing sebum secretion.

8. At least one selected from the group consisting of siRNAs for each of HIF1α gene, ENO1 gene, LDHA gene, PGK1 gene, GPI gene, and HK1 gene for use in
(i) the prevention or improvement of acne;
(ii) the prevention or improvement of alopecia; or
(iii) the prevention or improvement of hypertrichosis.

## Patentansprüche

1. *In* vitro-Verfahren zum Beurteilen oder Auswählen eines Talgdrüsen- oder Haarfollikel-selektiven Regulators der Androgenrezeptor-Aktivität, wobei das Verfahren die Schritte umfasst:
(A) Aufbringen eines Testagens auf Zellen von Talgdrüsen oder Haarfollikeln unter hypoxischen Bedingungen;
(B') Messen der Aktivität eines durch HIF1α in den Zellen induzierten Faktors, wobei der durch HIF1α induzierte Faktor ein glycolytisches Enzym ist;
(C') Vergleichen der in (B') gemessenen Aktivität mit der Aktivität des Faktors in einer Kontrollgruppe; und
(D') Bestimmen einer regulatorischen Wirkung des Testagens auf die Aktivität des Faktors, basierend auf dem Ergebnis in (C').

2. Verfahren nach Anspruch 1, das des Weiteren umfasst:
(E') Auswählen des Testagens, von dem in (D') nachgewiesen wurde, dass es eine verringernde Wirkung auf die Aktivität des durch HIF1α induzierten Faktors hat, als ein Talgdrüsen- oder Haarfollikel-selektiver Repressor der Androgenrezeptor-Aktivität, ein Repressor von Talgsekretion, ein Agens zur Vorbeugung oder Verbesserung von Akne, ein Agens zur Vorbeugung oder Verbesserung von Alopezie, oder ein Agens zur Vorbeugung oder Verbesserung von Hypertrichose.

3. Verfahren nach Anspruch 1, das des Weiteren umfasst:
(E') Auswählen des Testagens, von dem in (D') nachgewiesen wurde, dass es eine steigernde Wirkung auf die Aktivität des durch HIF1α induzierten Faktors hat, als ein Talgdrüsen- oder Haarfollikel-selektiver Aktivator des Androgenrezeptors, ein Aktivator von Talgsekretion, oder ein Agens zur Vorbeugung oder Verbesserung von seniler Xerose.

4. Verfahren nach einem der Ansprüche 1 bis 3, wobei das Messen der Aktivität des durch HIF1α induzierten Faktors das Messen eines Expressionsspiegels von mindestens einem Gen ist, das ausgewählt ist aus der Gruppe bestehend aus ENO1, LDHA, PGK1, GPI und HK1 oder eines Expressionsspiegels oder eines Spiegels enzymatischer Aktivität von mindestens einem Enzym, das ausgewählt ist aus der Gruppe bestehend aus Enzymen, die von diesen Genen codiert werden.

5. Verfahren nach einem der Ansprüche 1 bis 4, wobei die Zellen von Talgdrüsen oder Haarfollikeln vom Menschen sind.

6. Verfahren nach einem der Ansprüche 1 bis 5, wobei der hypoxische Zustand ein Zustand einer Sauerstoffkonzentration in einer Kulturatmosphäre von 5% oder weniger ist.

7. Nicht-therapeutische Verwendung von mindestens einem ausgewählt aus der Gruppe bestehend aus siRNAs für jedes HIF1α-Gen, ENO1-Gen, LDHA-Gen, PGK1-Gen, GPI-Gen und HK1-Gen zur Verwendung bei der Unterdrückung von Talgsekretion.

8. Mindestens eines ausgewählt aus der Gruppe bestehend aus siRNAs für jedes HIF1α-Gen, ENO1-Gen, LDHA-Gen, PGK1-Gen, GPI-Gen und HK1-Gen zur Verwendung bei
(i) der Vorbeugung oder Verbesserung von Akne;
(ii) der Vorbeugung oder Verbesserung von Alopezie; oder
(iii) der Vorbeugung oder Verbesserung von Hypertrichose.

## Revendications

1. Méthode in vitro d'évaluation ou de sélection d'un régulateur de l'activité de récepteur d'androgène sélectif vis-à-vis des glandes sébacées ou des follicules pileux, la méthode comprenant les étapes :
(A) d'application d'un agent de test à des cellules provenant de glandes sébacées ou de follicules pileux dans des conditions hypoxiques ;
(B') de mesure de l'activité d'un facteur induit par HIF1α dans les cellules, dans lequel le facteur induit par RIF1α est une enzyme glycolytique ;
(C') de comparaison de l'activité mesurée en (B') avec l'activité du facteur dans un groupe témoin ; et
(D') de détermination de l'effet régulateur de l'agent de test sur l'activité du facteur sur la base du résultat en (C').

2. Méthode selon la revendication 1, comprenant en outre :
(E') la sélection de l'agent de test qui est déterminé avoir un effet de réduction de l'activité du facteur induit par RIF1α en (D') en tant que répresseur de l'activité de récepteur d'androgène sélectif vis-à-vis des glandes sébacées ou des follicules pileux, répresseur de la sécrétion de sébum, agent pour prévenir ou améliorer l'acné, agent pour prévenir ou améliorer l'alopécie, ou agent pour prévenir ou améliorer l'hypertrichose.

3. Méthode selon la revendication 1, comprenant en outre :
(E') la sélection de l'agent de test qui est déterminé avoir un effet d'augmentation de l'activité du facteur induit par RIF1α en (D') en tant qu'activateur de récepteur d'androgène sélectif vis-à-vis des glandes sébacées ou des follicules pileux, activateur de la sécrétion de sébum, ou agent pour prévenir ou améliorer la xérose sénile.

4. Méthode selon l'une quelconque des revendications 1 à 3, dans laquelle la mesure de l'activité du facteur induit par RIF1α est la mesure du niveau d'expression d'au moins un gène choisi dans le groupe constitué par ENO1, LDHA, PGK1, GPI et HK1 ou du niveau d'expression ou du niveau d'activité enzymatique d'au moins une enzyme choisie dans le groupe constitué par les enzymes codées par ces gènes.

5. Méthode selon l'une quelconque des revendications 1 à 4, dans laquelle les cellules provenant de glandes sébacées ou de follicules pileux proviennent d'un humain.

6. Méthode selon l'une quelconque des revendications 1 à 5, dans laquelle la condition hypoxique est une condition de concentration d'oxygène dans l'atmosphère de culture de 5 % ou moins.

7. Utilisation non thérapeutique d'au moins l'un choisi dans le groupe constitué par les ARNsi pour chacun parmi le gène HIF1α, le gène ENO1, le gène LDHA, le gène PGK1, le gène GPI, et le gène HK1, destiné à être utilisé dans la répression de la sécrétion de sébum.

8. Au moins l'un choisi dans le groupe constitué par les ARNsi pour chacun parmi le gène HIF1α, le gène ENO1, le gène LDHA, le gène PGK1, le gène GPI, et le gène HK1, pour son utilisation dans
(i) la prévention ou l'amélioration de l'acné ;
(ii) la prévention ou l'amélioration de l'alopécie ; ou
(iii) la prévention ou l'amélioration de l'hypertrichose.


### SEQUENCE LISTING

<110> Kao Corporation
<120> Method of evaluating or selecting androgen receptor regulator
   specific to sebaceous gland in skin or hair follicle
<130> AA1688 EP S3
<160> 10
<170> PatentIn version 3.5
<210> 1
   <211> 2481
   <212> DNA
   <213> Homo sapiens
<400> 1
<210> 2
   <211> 826
   <212> PRT
   <213> Homo sapiens
<400> 2
<210> 3
   <211> 1305
   <212> DNA
   <213> Homo sapiens
<400> 3
   atgtctattc tcaagatcca tgccagggag atctttgact ctcgcgggaa tcccactgtt      60
   gaggttgatc tcttcacctc aaaaggtctc ttcagagctg ctgtgcccag tggtgcttca     120
   actggtatct atgaggccct agagctccgg gacaatgata agactcgcta tatggggaag     180
   ggtgtctcaa aggctgttga gcacatcaat aaaactattg cgcctgccct ggttagcaag     240
   aaactgaacg tcacagaaca agagaagatt gacaaactga tgatcgagat ggatggaaca     300
   gaaaataaat ctaagtttgg tgcgaacgcc attctggggg tgtcccttgc cgtctgcaaa     360
   gctggtgccg ttgagaaggg ggtccccctg taccgccaca tcgctgactt ggctggcaac      420
   tctgaagtca tcctgccagt cccggcgttc aatgtcatca atggcggttc tcatgctggc     480
   aacaagctgg ccatgcagga gttcatgatc ctcccagtcg gtgcagcaaa cttcagggaa     540
   gccatgcgca ttggagcaga ggtttaccac aacctgaaga atgtcatcaa ggagaaatat     600
   gggaaagatg ccaccaatgt gggggatgaa ggcgggtttg ctcccaacat cctggagaat     660
   aaagaaggcc tggagctgct gaagactgct attgggaaag ctggctacac tgataaggtg     720
   gtcatcggca tggacgtagc ggcctccgag ttcttcaggt ctgggaagta tgacctggac     780
   ttcaagtctc ccgatgaccc cagcaggtac atctcgcctg accagctggc tgacctgtac     840
   aagtccttca tcaaggacta cccagtggtg tctatcgaag atccctttga ccaggatgac     900
   tggggagctt ggcagaagtt cacagccagt gcaggaatcc aggtagtggg ggatgatctc     960
   acagtgacca acccaaagag gatcgccaag gccgtgaacg agaagtcctg caactgcctc    1020
   ctgctcaaag tcaaccagat tggctccgtg accgagtctc ttcaggcgtg caagctggcc    1080
   caggccaatg gttggggcgt catggtgtct catcgttcgg gggagactga agataccttc    1140
   atcgctgacc tggttgtggg gctgtgcact gggcagatca agactggtgc cccttgccga    1200
   tctgagcgct tggccaagta caaccagctc ctcagaattg aagaggagct gggcagcaag    1260
   gctaagtttg ccggcaggaa cttcagaaac cccttggcca agtaa                    1305
<210> 4
   <211> 999
   <212> **DNA**
   <213> Homo sapiens
<400> 4
   atggcaactc taaaggatca gctgatttat aatcttctaa aggaagaaca gaccccccag      60
   aataagatta cagttgttgg ggttggtgct gttggcatgg cctgtgccat cagtatctta     120
   atgaaggact tggcagatga acttgctctt gttgatgtca tcgaagacaa attgaaggga     180
   gagatgatgg atctccaaca tggcagcctt ttccttagaa caccaaagat tgtctctggc     240
   aaagactata atgtaactgc aaactccaag ctggtcatta tcacggctgg ggcacgtcag     300
   caagagggag aaagccgtct taatttggtc cagcgtaacg tgaacatctt taaattcatc     360
   attcctaatg ttgtaaaata cagcccgaac tgcaagttgc ttattgtttc aaatccagtg     420
   gatatcttga cctacgtggc ttggaagata agtggttttc ccaaaaaccg tgttattgga     480
   agcggttgca atctggattc agcccgattc cgttacctaa tgggggaaag gctgggagtt     540
   cacccattaa gctgtcatgg gtgggtcctt ggggaacatg gagattccag tgtgcctgta     600
   tggagtggaa tgaatgttgc tggtgtctct ctgaagactc tgcacccaga tttagggact     660
   gataaagata aggaacagtg gaaagaggtt cacaagcagg tggttgagag tgcttatgag     720
   gtgatcaaac tcaaaggcta cacatcctgg gctattggac tctctgtagc agatttggca     780
   gagagtataa tgaagaatct taggcgggtg cacccagttt ccaccatgat taagggtctt     840
   tacggaataa aggatgatgt cttccttagt gttccttgca ttttgggaca gaatggaatc     900
   tcagaccttg tgaaggtgac tctgacttct gaggaagagg cccgtttgaa gaagagtgca     960
   gatacacttt gggggatcca aaaggagctg caattttaa                            999
<210> 5
   <211> 1170
   <212> DNA
   <213> Homo sapiens
<400> 5
   atgaagaaca accagataac aaacaaccag aggattaagg ctgctgtccc aagcatcaaa      60
   ttctgcttgg acaatggagc caagtcggta gtccttatga gccacctagg ccggcctgat     120
   ggtgtgccca tgcctgacaa gtactcctta gagccagttg ctgtagaact caaatctctg     180
   ctgggcaagg atgttctgtt cttgaaggac tgtgtaggcc cagaagtgga gaaagcctgt     240
   gccaacccag ctgctgggtc tgtcatcctg ctggagaacc tccgctttca tgtggaggaa     300
   gaagggaagg gaaaagatgc ttctgggaac aaggttaaag ccgagccagc caaaatagaa     360
   gctttccgag cttcactttc caagctaggg gatgtctatg tcaatgatgc ttttggcact     420
   gctcacagag cccacagctc catggtagga gtcaatctgc cacagaaggc tggtgggttt     480
   ttgatgaaga aggagctgaa ctactttgca aaggccttgg agagcccaga gcgacccttc     540
   ctggccatcc tgggcggagc taaagttgca gacaagatcc agctcatcaa taatatgctg     600
   gacaaagtca atgagatgat tattggtggt ggaatggctt ttaccttcct taaggtgctc     660
   aacaacatgg agattggcac ttctctgttt gatgaagagg gagccaagat tgtcaaagac     720
   ctaatgtcca aagctgagaa gaatggtgtg aagattacct tgcctgttga ctttgtcact     780
   gctgacaagt ttgatgagaa tgccaagact ggccaagcca ctgtggcttc tggcatacct     840
   gctggctgga tgggcttgga ctgtggtcct gaaagcagca agaagtatgc tgaggctgtc     900
   actcgggcta agcagattgt gtggaatggt cctgtggggg tatttgaatg ggaagctttt     960
   gcccggggaa ccaaagctct catggatgag gtggtgaaag ccacttctag gggctgcatc     1020
   accatcatag gtggtggaga cactgccact tgctgtgcca aatggaacac ggaggataaa    1080
   gtcagccatg tgagcactgg gggtggtgcc agtttggagc tcctggaagg taaagtcctt    1140
   cctggggtgg atgctctcag caatatttag                                     1170
<210> 6
   <211> 1710
   <212> **DNA**
   <213> Homo sapiens
<400> 6
   atggtagctc tctgcagcct ccaacacctg ggctccagtg atccccgggc tctgcccacc      60
   ctccccactg ccacttccgg gcagaggcca gcaaagcggc ggcgcaagag tcccgccatg     120
   gccgctctca cccgggaccc ccagttccag aagctgcagc aatggtaccg cgagcaccgc     180
   tccgagctga acctgcgccg cctcttcgat gccaacaagg accgcttcaa ccacttcagc     240
   ttgaccctca acaccaacca tgggcatatc ctggtggatt actccaagaa cctggtgacg     300
   gaggacgtga tgcggatgct ggtggacttg gccaagtcca ggggcgtgga ggccgcccgg     360
   gagcggatgt tcaatggtga gaagatcaac tacaccgagg gtcgagccgt gctgcacgtg     420
   gctctgcgga accggtcaaa cacacccatc ctggtagacg gcaaggatgt gatgccagag     480
   gtcaacaagg ttctggacaa gatgaagtct ttctgccagg gacccctcat ggtgactgaa     540
   gcccttaagc catactcttc aggaggtccc cgcgtctggt atgtctccaa cattgatgga     600
   actcacattg ccaaaaccct ggcccagctg aaccccgagt cctccctgtt catcattgcc     660
   tccaagacct ttactaccca ggagaccatc acgaatgcag agacggcgaa ggagtggttt     720
   ctccaggcgg ccaaggatcc ttctgcagtg gcgaagcact ttgttgccct gtctactaac     780
   acaaccaaag tgaaggagtt tggaattgac cctcaaaaca tgttcgagtt ctgggattgg     840
   gtgggaggac gctactcgct gtggtcggcc atcggactct ccattgccct gcacgtgggt     900
   tttgacaact tcgagcagct gctctcgggg gctcactgga tggaccagca cttccgcacg     960
   acgcccctgg agaagaacgc ccccgtcttg ctggccctgc tgggtatctg gtacatcaac    1020
   tgctttgggt gtgagacaca cgccatgctg ccctatgacc agtacctgca ccgctttgct    1080
   gcgtacttcc agcagggcga catggagtcc aatgggaaat acatcaccaa atctggaacc    1140
   cgtgtggacc accagacagg ccccattgtg tggggggagc cagggaccaa tggccagcat    1200
   gctttttacc agctcatcca ccaaggcacc aagatgatac cctgtgactt cctcatcccg    1260
   gtccagaccc agcaccccat acggaagggt ctgcatcaca agatcctcct ggccaacttc    1320
   ttggcccaga cagaggccct gatgagggga aaatcgacgg aggaggcccg aaaggagctc    1380
   caggctgcgg gcaagagtcc agaggacctt gagaggctgc tgccacataa ggtctttgaa    1440
   ggaaatcgcc caaccaactc tattgtgttc accaagctca caccattcat gcttggagcc    1500
   ttggtcgcca tgtatgagca caagatcttc gttcagggca tcatctggga catcaacagc    1560
   tttgaccagt ggggagtgga gctgggaaag cagctggcta agaaaataga gcctgagctt    1620
   gatggcagtg ctcaagtgac ctctcacgac gcttctacca atgggctcat caacttcatc    1680
   aagcagcagc gcgaggccag agtccaataa                                     1710
<210> 7
   <211> 2754
   <212> **DNA**
   <213> Homo sapiens
<400> 7
   atgatcgccg cgcagctcct ggcctattac ttcacggagc tgaaggatga ccaggtcaaa      60
   aagattgaca agtatctcta tgccatgcgg ctctccgatg aaactctcat agatatcatg     120
   actcgcttca ggaaggagat gaagaatggc ctctcccggg attttaatcc aacagccaca     180
   gtcaagatgt tgccaacatt cgtaaggtcc attcctgatg gctctgaaaa gggagatttc     240
   attgccctgg atcttggtgg gtcttccttt cgaattctgc gggtgcaagt gaatcatgag     300
   aaaaaccaga atgttcacat ggagtccgag gtttatgaca ccccagagaa catcgtgcac     360
   ggcagtggaa gccagctttt tgatcatgtt gctgagtgcc tgggagattt catggagaaa     420
   aggaagatca aggacaagaa gttacctgtg ggattcacgt tttcttttcc ttgccaacaa     480
   tccaaaatag atgaggccat cctgatcacc tggacaaagc gatttaaagc gagcggagtg     540
   gaaggagcag atgtggtcaa actgcttaac aaagccatca aaaagcgagg ggactatgat     600
   gccaacatcg tagctgtggt gaatgacaca gtgggcacca tgatgacctg tggctatgac     660
   gaccagcact gtgaagtcgg cctgatcatc ggcactggca ccaatgcttg ctacatggag     720
   gaactgaggc acattgatct ggtggaagga gacgagggga ggatgtgtat caatacagaa     780
   tggggagcct ttggagacga tggatcatta gaagacatcc ggacagagtt tgacagggag     840
   atagaccggg gatccctcaa ccctggaaaa cagctgtttg agaagatggt cagtggcatg     900
   tacttgggag agctggttcg actgatccta gtcaagatgg ccaaggaggg cctcttattt     960
   gaagggcgga tcaccccgga gctgctcacc cgagggaagt ttaacaccag tgatgtgtca    1020
   gccatcgaaa agaataagga aggcctccac aatgccaaag aaatcctgac ccgcctggga    1080
   gtggagccgt ccgatgatga ctgtgtctca gtccagcacg tttgcaccat tgtctcattt    1140
   cgctcagcca acttggtggc tgccacactg ggcgccatct tgaaccgcct gcgtgataac 1200
   aagggcacac ccaggctgcg gaccacggtt ggtgtcgacg gatctcttta caagacgcac 1260
   ccacagtatt cccggcgttt ccacaagact ctaaggcgct tggtgccaga ctccgatgtg 1320
   cgcttcctcc tctcggagag tggcagcggc aagggggctg ccatggtgac ggcggtggcc 1380
   taccgcttgg ccgagcagca ccggcagata gaggagaccc tggctcattt ccacctcacc 1440
   aaggacatgc tgctggaggt gaagaagagg atgcgggccg agatggagct ggggctgagg 1500
   aagcagacgc acaacaatgc cgtggttaag atgctgccct ccttcgtccg gagaactccc 1560
   gacgggaccg agaatggtga cttcttggcc ctggatcttg gaggaaccaa tttccgtgtg 1620
   ctgctggtga aaatccgtag tgggaaaaag agaacggtgg aaatgcacaa caagatctac 1680
   gccattccta ttgaaatcat gcagggcact ggggaagagc tgtttgatca cattgtctcc 1740
   tgcatctctg acttcttgga ctacatgggg atcaaaggcc ccaggatgcc tctgggcttc 1800
   acgttctcat ttccctgcca gcagacgagt ctggacgcgg gaatcttgat cacgtggaca 1860
   aagggtttta aggcaacaga ctgcgtgggc cacgatgtag tcaccttact aagggatgcg 1920
   ataaaaagga gagaggaatt tgacctggac gtggtggctg tggtcaacga cacagtgggc 1980
   accatgatga cctgtgctta tgaggagccc acctgtgagg ttggactcat tgttgggacc 2040
   ggcagcaatg cctgctacat ggaggagatg aagaacgtgg agatggtgga gggggaccag 2100
   gggcagatgt gcatcaacat ggagtggggg gcctttgggg acaacgggtg tctggatgat 2160
   atcaggacac actacgacag actggtggac gaatattccc taaatgctgg gaaacaaagg 2220
   tatgagaaga tgatcagtgg tatgtacctg ggtgaaatcg tccgcaacat cttaatcgac 2280
   ttcaccaaga agggattcct cttccgaggg cagatctctg agacgctgaa gacccggggc 2340
   atctttgaga ccaagtttct ctctcagatc gagagtgacc gattagcact gctccaggtc 2400
   cgggctatcc tccagcagct aggtctgaat agcacctgcg atgacagtat cctcgtcaag 2460
   acagtgtgcg gggtggtgtc caggagggcc gcacagctgt gtggcgcagg catggctgcg 2520
   gttgtggata agatccgcga gaacagagga ctggaccgtc tgaatgtgac tgtgggagtg 2580
   gacgggacac tctacaagct tcatccacac ttctccagaa tcatgcacca gacggtgaag 2640
   gaactgtcac caaaatgtaa cgtgtccttc ctcctgtctg aggatggcag cggcaagggg 2700
   gccgccctca tcacggccgt gggcgtgcgg ttacgcacag aggcaagcag ctaa 2754
<210> 8
   <211> 25
   <212> RNA
   <213> Homo sapiens
<220>
   <223> siRNA for HIF1-alpha gene
<400> 8
   ccagccgcug gagacacaau cauau 25
<210> 9
   <211> 25
   <212> RNA
   <213> Homo sapiens
<220>
   <223> siRNA for HIF1-alpha gene
<400> 9
   gggauuaacu caguuugaac uaacu 25
<210> 10
   <211> 25
   <212> RNA
   <213> Homo sapiens
<220>
   <223> siRNA for HIF1-alpha gene
<400> 10
   gaaauuccuu uagauagcaa gacuu 25